# FASCICULE DE BREVET EUROPEEN

(11) **EP 0 559 502 B1**
(45) Date de publication et mention de la délivrance du brevet: **20.03.1996**
(21) Numéro de dépôt: 93400195.9
(22) Date de dépôt: 27.01.1993
(51) Int. Cl.: A61K 7/00

(54) **Composition pour traitement topique contenant des vésicules lipidiques encapsulant au moins une eau minérale**
Topisches Behandlungsmittel enthaltend Lipidvesikeln die mindestens ein Mineralwasser einkapseln
Topical care composition containing lipidic vesicles encapsulating at least one mineral water

(30) Priorité: 28.02.1992 FR 9202372
(43) Date de publication de la demande: 08.09.1993
(73) Titulaire: L'OREAL, F-75008 Paris (FR)
(72) Inventeur: Laugier, Jean-Pierre, F-92160 Antony (FR); Ribier, Alain, F-75014 Paris (FR); Richard, Alain, F-86270 La Roche Posay (FR); Brissonnet, Jean-Pierre, F-86000 Poitiers (FR)
(74) Mandataire: Peuscet, Jacques

(56) Documents cités:
- EP-A- 0 274 363
- WO-A-92/06666
- PATENT ABSTRACTS OF JAPAN vol. 8, no. 126 (C-228)13 Juin 1984

## Description

L'invention concerne une composition constituée par une dispersion de vésicules à structure lamellaire, dont les membranes sont constituées d'au moins un lipide amphiphile, lesdites vésicules encapsulant au moins une eau minérale, ainsi que son utilisation therapeutique pour le traitement topique de la peau ou des ongles.

On sait que de nombreuses eaux minérales sont douées d'actions thérapeutiques importantes sur des dermatoses. On peut citer notamment, à titre d'exemples, les eaux d'AX LES THERMES, de LA BOURBOULE, de LUCHON, de MOLITG LES BAINS, de LA ROCHE-POSAY, de SAINT-CHRISTAU, et d'AVENE, toutes ces stations thermales étant situées sur le territoire français. Ces eaux thermales ont en commun le fait qu'elles sont silicatées et riches en oligoéléments tels que Zn, Cu, Ni, Li, Sr, Fe, Mn, B, F, Co, As et Se. Leurs propriétés thérapeutiques sont directement reliées à la présence de certains des oligoéléments susmentionnés et à la présence de silice sous forme de silicates naturels ; la teneur en silice peut varier de 1 à 150 mg/l. La résistivité de ces diverses eaux minérales varie entre 50 et 5 000 ohms.cm⁻¹.

On a donc considéré qu'il était souhaitable d'utiliser, dans des compositions cosmétiques, des eaux minérales même non exploitées à ce jour à des fins thérapeutiques en dermatologie, notamment pour traiter certaines affections de la peau pathologiques ou pour maintenir en état des peaux agressées ou abîmées.

C'est ainsi que l'on a propose dans la demande de brevet FR-A-2 546 754 des agents cosmétiques pour l'entretien de la santé et du corps, caractérisés en ce qu'ils contiennent des eaux minérales natives, des eaux curatives ou leurs mélanges ; ces agents cosmétiques peuvent contenir, en outre, des protéines, dont l'action est associée aux oligoéléments des eaux minérales pour la régénération de la peau et le traitement de dermatoses, telles que brûlure, eczéma, ou psoriasis, la protéine semblant agir comme transporteur des oligoéléments.

On a également proposé dans le brevet FR-B-2 608 426 un procédé pour effectuer un traitement hydratant de la peau au moyen de liposomes contenant de l'eau minérale ; ces liposomes sont obtenus essentiellement à partir de lipides amphiphiles ioniques, à savoir les phospholipides. Les compositions proposées peuvent éventuellement contenir également des glucosaminoglycanes, qui ont par eux-mêmes un effet hydratant. Etant donne que les exemples de ce brevet mettent toujours en oeuvre les glucosaminoglycanes en même temps que l'eau minérale liposomée, il n'est pas possible pour l'homme de métier de déterminer si le conditionnement liposomique de l'eau minérale est ou non réellement responsable de l'effet hydratant constaté.

On sait, en outre, que certaines eaux thermales, notamment l'eau thermale de LA ROCHE-POSAY, ont des propriétés antiradicalaires vis-à-vis des radicaux libres oxygénés impliques dans le vieillissement cellulaire ; on a démontré, par des techniques de culture de fibroblastes humains, qu'une eau thermale séléniée riche en oligoéléments, tels que le Zn ou le Cu, présentait un pouvoir antiradicalaire (M.J.RICHARD et autres, "LES NOUVELLES DERMATOLOGIQUES", volume 9, n° 3, mars 1990) ; on a montré également que l'application percutanée d'eau thermale de LA ROCHE-POSAY protége la peau contre la peroxydation lipidique photoinduite (R. CADI et autres, "LES NOUVELLES DERMATOLOGIQUES", volume 10, n° 3, mars 1991).

La société déposante a maintenant découvert que, de façon surprenante, l'eau thermale naturelle, quand elle était formulée dans des vésicules obtenues à base de lipides amphiphiles non-ioniques, avait une efficacité plus grande que lorsqu'elle était formulée dans des vésicules obtenues à base de lipides amphiphiles ioniques, notamment en ce qui concerne son pouvoir antioxydant et son activité antiradicalaire. Cette potentialisation améliorée des effets d'une eau minérale due à sa formulation par encapsulation dans des vésicules lipidiques de lipides amphiphiles non-ioniques n'était aucunement suggérée à l'homme de métier par les documents de l'état de la technique.

Lorsqu'une composition selon l'invention est utilisée pour le traitement des ongles, l'application d'une eau minérale à pouvoir anti-oxydant permet d'éviter la peroxydation des lipides des ongles et, par conséquent, le jaunissement des ongles.

La présente invention a, en conséquence, pour objet le produit industriel nouveau que constitue une composition pour le traitement topique de la peau ou des ongles comportant dans une phase aqueuse de dispersion, des vésicules délimitées par une membrane de phase lipidique contenant au moins un lipide amphiphile, lesdites vésicules contenant une phase encapsulée au moins partiellement constituée d'au moins une eau minérale formant un actif pour le traitement de la peau, caractérisée par le fait que la phase lipidique membranaire des vésicules contient au moins un lipide amphiphile non-ionique choisi dans le groupe formé par :
(1) les dérivés du glycérol, linéaires ou ramifiés, de formule 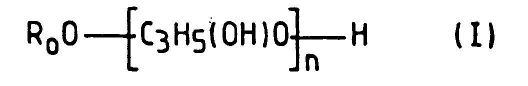 formule (I) dans laquelle :
   . -C₃H₅(OH)O-
      est représenté par les structures suivantes prises en mélange ou séparément :
      -CH₂CHOHCH₂O- , 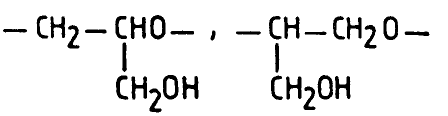
   . n est une valeur statistique moyenne comprise entre 1 et 6 ou bien n = 1 ou 2, auquel cas -C₃H₅(OH)O- est représenté par la structure -CH₂-CHOH-CH₂O ;
   . Rₒ représente :
      (a) une chaîne aliphatique, linéaire ou ramifiée saturée ou insaturée, contenant de 12 à 30 atomes de carbone ; ou des radicaux hydrocarbonés des alcools de lanoline ; ou les restes d'alpha-diols à longue chaîne ;
      (b) un reste R₁CO, où R₁ est un radical aliphatique, linéaire ou ramifié, en C₁₁-C₂₉ ;
      (c) un reste R₂⁅OC₂H₃(R₃)⁆
         où :
         . R₂ peut prendre la signification (a) ou (b) donnée pour Rₒ ;
         . -OC₂H₃(R₃)- est représenté par les structures suivantes, prises en mélange ou séparément : 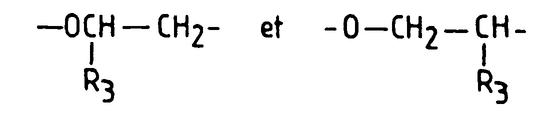
   où R₃ prend la signification (a) donnée pour Rₒ ;
(2) les éthers de polyglycérol, linéaires ou ramifiés, comportant deux chaînes grasses ;
(3) les diols à chaîne grasse ;
(4) les alcools gras, oxyéthylénés ou non, les stérols, tels que le β-sitostérol, le cholestérol, et les phytostérols, oxyéthylénés ou non ;
(5) les éthers et esters de polyols, oxyéthylénés ou non, l'enchaînement des oxydes d'éthylène pouvant être linéaire ou cyclique ;
(6) les éthers et esters de mono ou polysaccharides et, notamment, les éthers et les esters de glucose ;
(7) les hydroxylamides représentés par la formule : 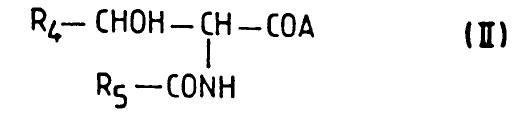 formule (II) dans laquelle :
   - R₄ désigne un radical alkyle ou alcényle en C₇-C₂₁ ;
   - R₅ désigne un radical hydrocarboné, saturé ou insaturé, en C₇-C₃₁ ;
   - COA désigne un groupement choisi parmi les deux groupements suivants :
      . un reste 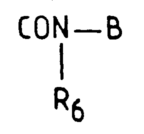 où :
      . B est un radical alcoyle dérivé d'amines primaires ou secondaires, mono- ou polyhydroxylées ; et
      . R₆ désigne un atome d'hydrogène ou un radical méthyle, éthyle ou hydroxyéthyle ; et
      . un reste -COOZ, où Z représente le reste d'un polyol en C₃-C₇.
(8) les céramides naturels ou de synthèse ;
(9) les dihydroxyalkylamines, les amines grasses oxyéthylénées ;
(10) les dérivés du glycérol décrits dans la demande de brevet PCT n° 91/00889 déposée le 13 Novembre 1991 et répondant à la formule : 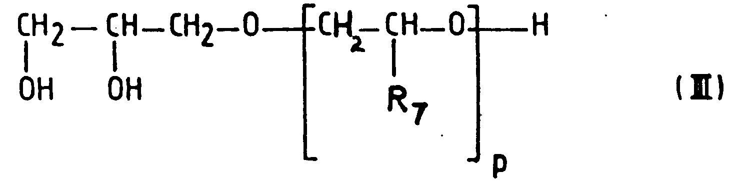 formule (III) dans laquelle R₇ représente un radical alkyle linéaire en C₁₄ à C₁₈ ou un groupement -CH₂A dans lequel A est -OR₁₄, R₁₄ représentant un radical alkyle linéaire en C₁₀-C₁₈ et, de préférence, en C₁₆, et p représente une valeur statistique moyenne supérieure à 1 et au plus égale à 3 et, en outre, lorsque R₇ = -CH₂A, p peut également représenter une valeur réelle (non statistique) égale à 2.

Dans une première variante de réalisation, le(s) lipide(s) amphiphile(s) de la phase lipidique membranaire est (sont tous) non-ionique(s) : on obtient des vésicules non-ioniques. Dans une deuxième variante de réalisation, les lipides amphiphiles de la phase lipidique membranaire sont constitues par un mélange de lipides non-ioniques et de lipides ioniques : on obtient des vésicules mixtes. La composition selon l'invention peut aussi contenir, outre les vésicules non-ioniques et/ou les vésicules mixtes, des vésicules dont la phase lipidique membranaire est constituée d'un ou plusieurs lipide(s) amphiphile(s) ionique(s).

Lorsque la phase lipidique des vésicules contient au moins un lipide amphiphile ionique, ce (ou ces) lipide(s) est (sont), de préférence, choisi(s), dans le groupe formé par :
(1) les lipides amphiphiles anioniques suivants :
   - les phospholipides naturels, les phospholipides modifiés par voie chimique ou enzymatique, et les phospholipides de synthèse ;
   - les composés anioniques de formule 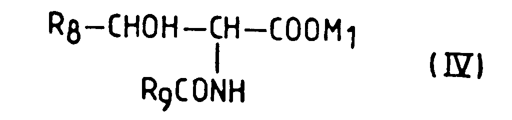 formule (IV) dans laquelle :
      . R₈ représente un radical alkyle ou alcényle en C₇-C₂₁
      . R₉ représente un radical hydrocarboné, saturé ou insaturé, en C₇-C₃₁, et
      . M₁ représente H, Na, K, NH₄ ou un ion ammonium substitué dérivé d'une amine ;
   - les composés anioniques, tels que les esters phosphoriques d'alcools gras notamment le dicétylphosphate et le dimyristylphosphate sous forme d'acides ou de sels alcalins ; l'acide heptylnonylbenzène sulfonique ; le sulfate de cholestérol acide ou le phosphate de cholestérol acide, ainsi que leurs sels alcalins ; les lysolécithines ; les alkylsulfates tels que le cétylsulfate de sodium ; les gangliosides ;
(2) les lipides amphiphiles cationiques suivants :
   - les composés cationiques ayant la formule : 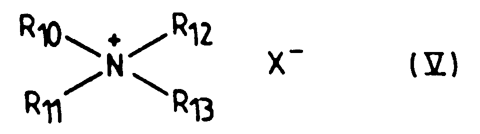 formule (V) dans laquelle R₁₀ et R₁₁, identiques ou différents, représentent des radicaux alkyle en C₁₂ - C₂₀ et R₁₂ et R₁₃, identiques ou différents, des radicaux alkyle en C₁ - C₄ ;
   - les amines à longue chaîne et leurs dérivés ammonium quaternaire ; les esters d'aminoalcools à longue chaîne et leurs sels et dérivés ammonium quaternaire ; et
   - les lipides polymérisables, comme décrits par RINGSDORF et autres, dans "ANGEVANDTE Chemic.", vol. 27, n° 1 Janvier 1988 pages 129-137.

On peut aussi ajouter à la phase lipidique constituant les parois vésiculaires des additifs, tels que certains polymères comme, par exemple, les polypeptides et les protéines.

La phase aqueuse de dispersion est, de préférence, choisie dans le groupe forme par l'eau et/ou l'eau minérale et les mélanges d'eau et/ou d'eau minérale avec au moins un alcool en C₁-C₇ et/ou un polyol d'alkyle en C₁-C₅. La phase aqueuse de dispersion peut également contenir des composes en solution, tels que des sucres, des sels organiques ou minéraux ou des polymères. La phase aqueuse de dispersion peut aussi contenir une dispersion de gouttelettes d'un liquide non miscible à l'eau que les vésicules stabilisent, de sorte qu'il n'est pas nécessaire d'introduire pour cette stabilisation un émulsionnant, tel que le monostéarate de glycérol par exemple ; ce liquide non miscible à l'eau peut être choisi dans le groupe formé par les huiles animales ou végétales, les huiles essentielles naturelles ou synthétiques, les hydrocarbures, les carbures halogénés, les silicones, les esters d'un acide minéral et d'un alcool, les éthers et les polyéthers.

Dans la composition selon l'invention, la phase lipidique totale de la dispersion représente avantageusement entre 0,01 % et 50 % en poids et, de préférence, entre 1 et 20 % en poids, par rapport au poids total de la dispersion. Le (ou les) lipide(s) amphiphile(s) représente(nt) avantageusement entre 10 et 95 % en poids et, de préférence, de 40 à 90 % en poids par rapport au poids total de la phase lipidique vesiculaire. Les vésicules ont, de préférence, des dimensions comprises entre 20 et 3 000 nanomètres, plus particulièrement entre 20 et 500 nanomètres.

Outre l'eau minérale, la composition selon l'invention peut également contenir au moins un actif à action cosmétique et/ou pharmaceutique ; dans le cas où cet actif est hydrosoluble, il peut être contenu dans la phase aqueuse de dispersion et/ou dans la phase aqueuse encapsulée dans les vésicules ; dans le cas où ledit actif est liposoluble, il est contenu dans la phase lipidique des vésicules. Des actifs amphiphiles peuvent aussi se repartir entre la phase aqueuse encapsulée et/ou de dispersion et la phase lipidique des vésicules.

Une liste non limitative des actifs utilisables dans les compositions selon l'invention est donnée dans le tableau I ci-après :

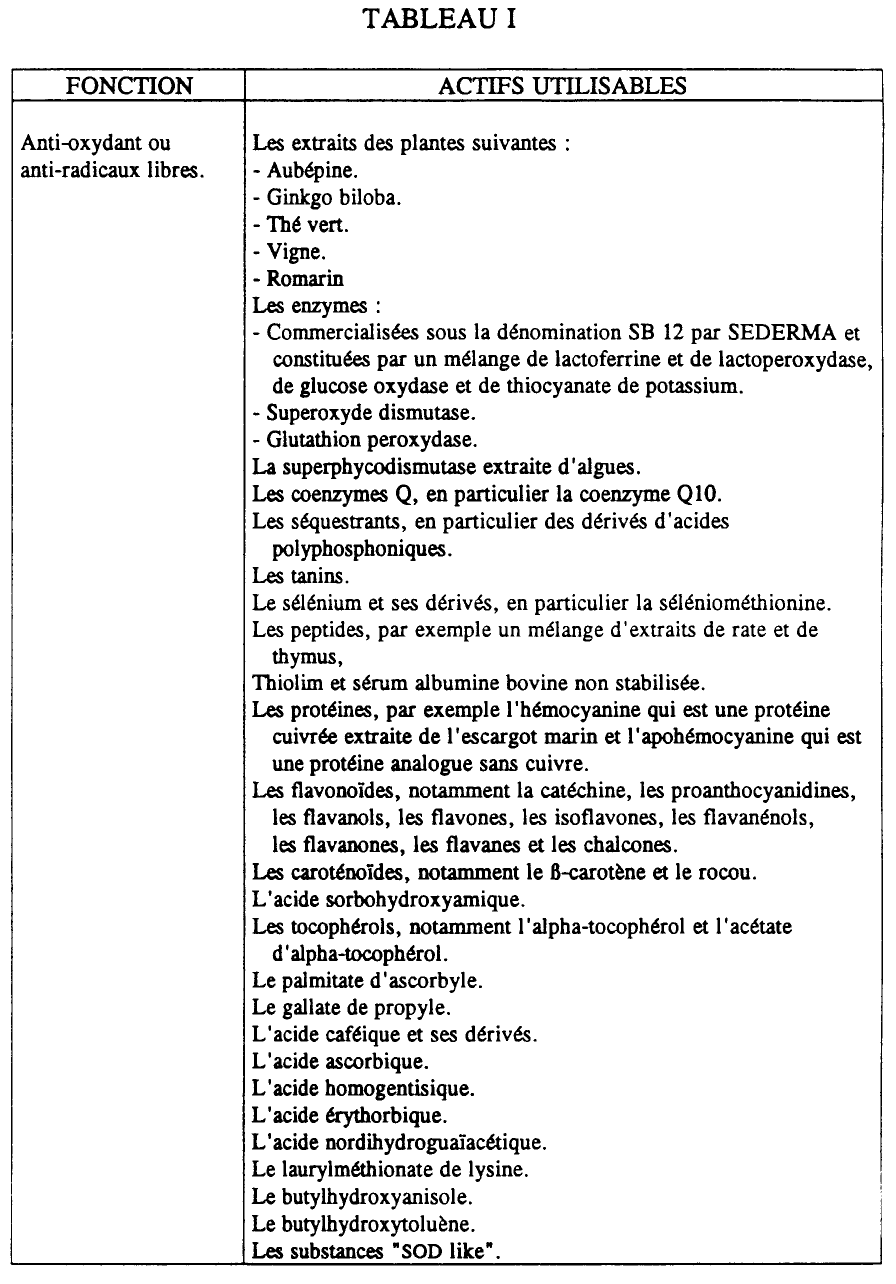

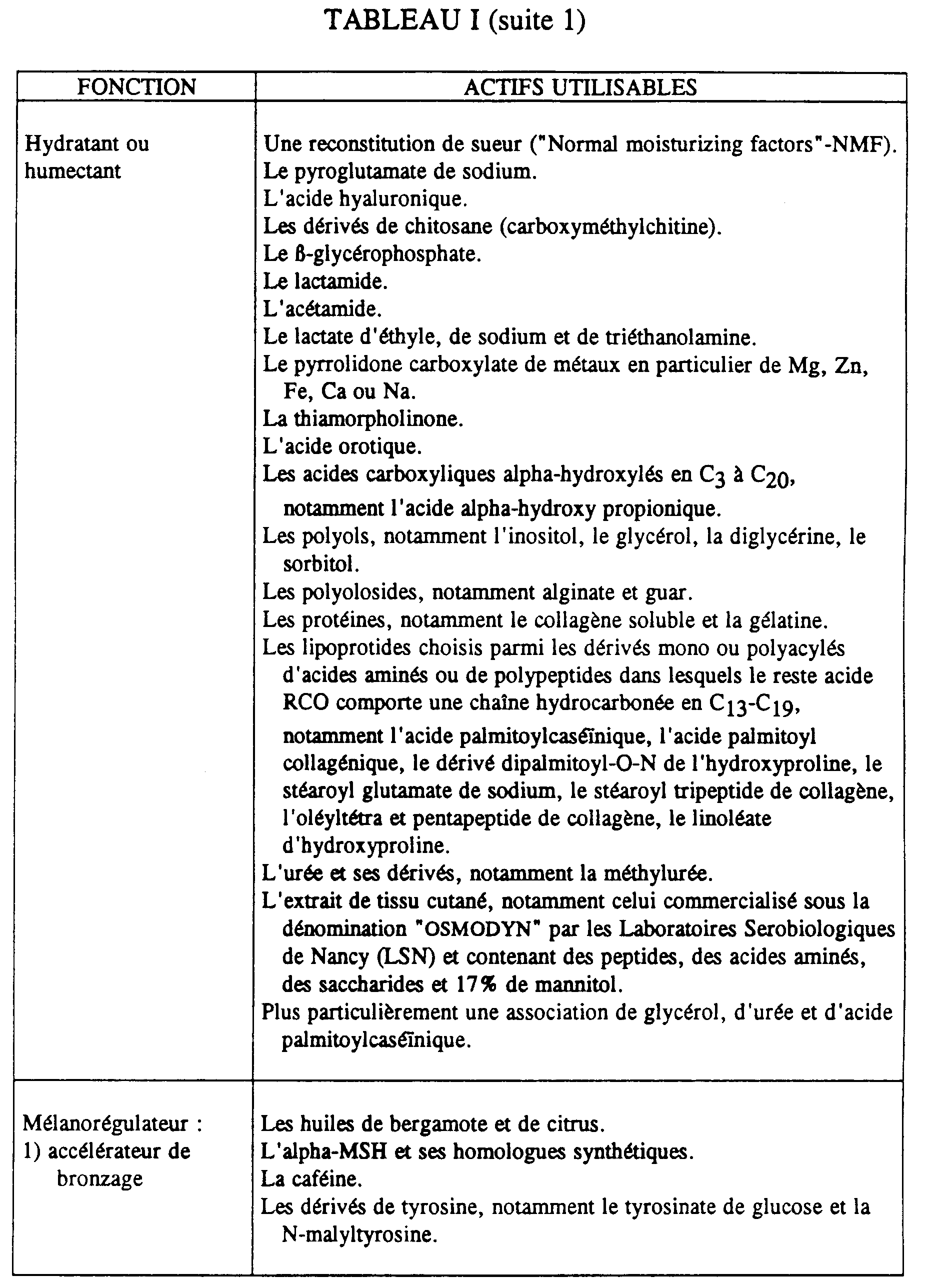

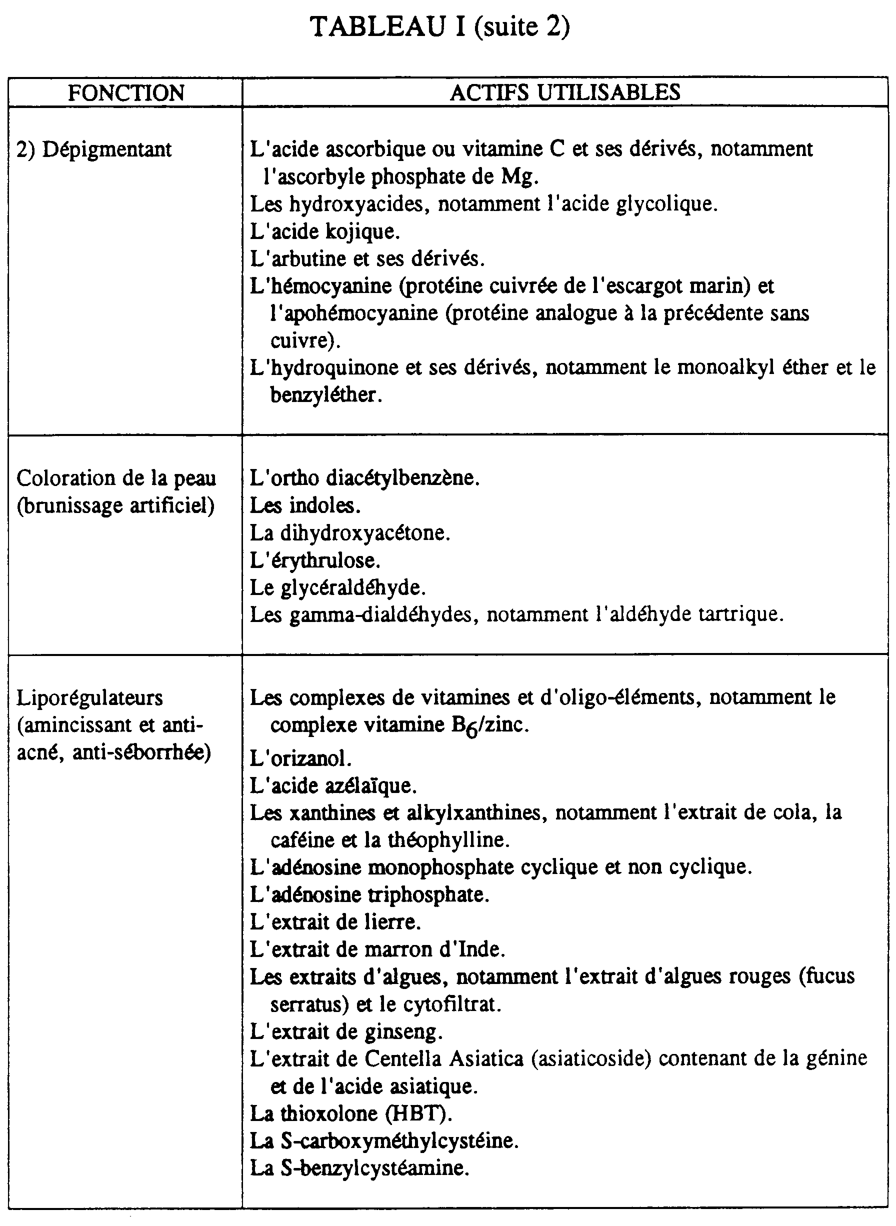

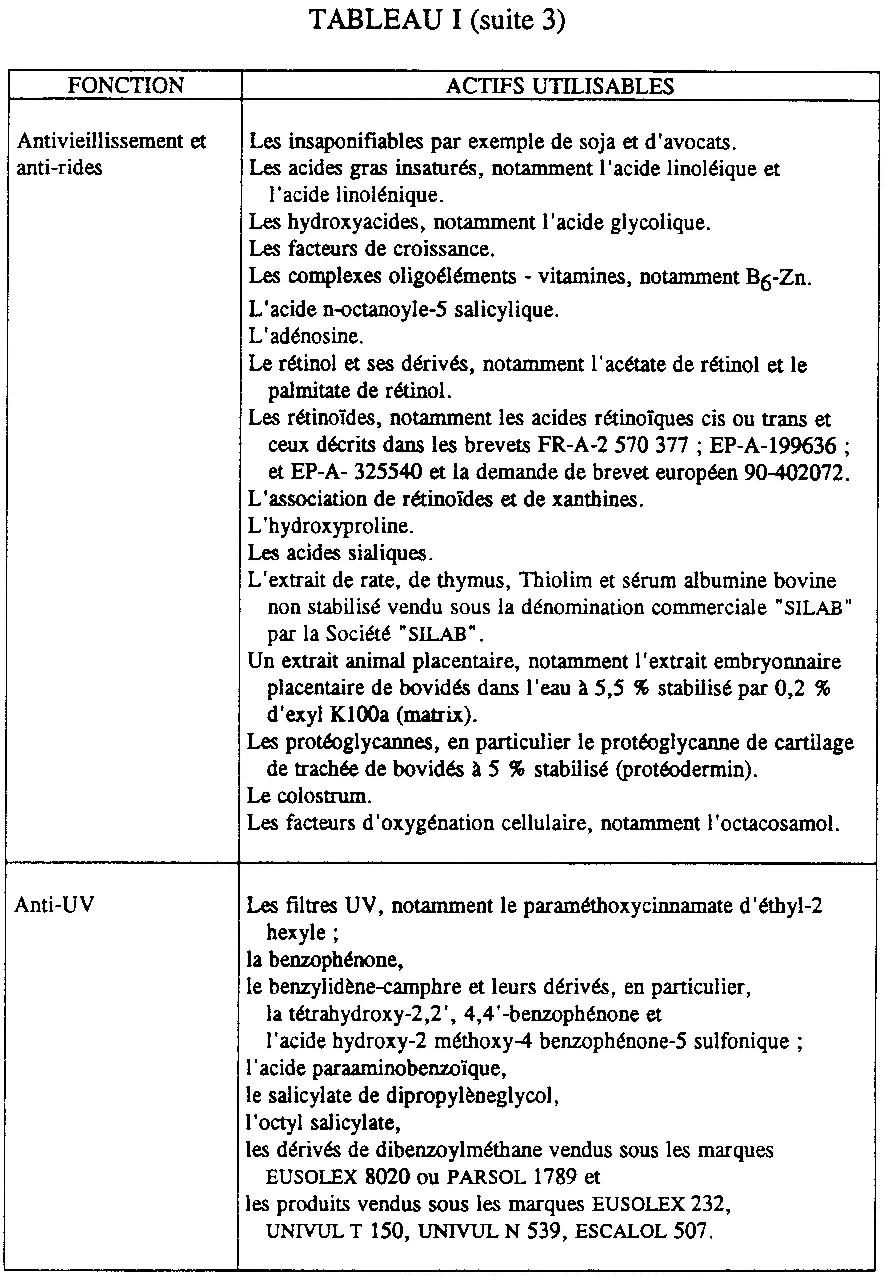

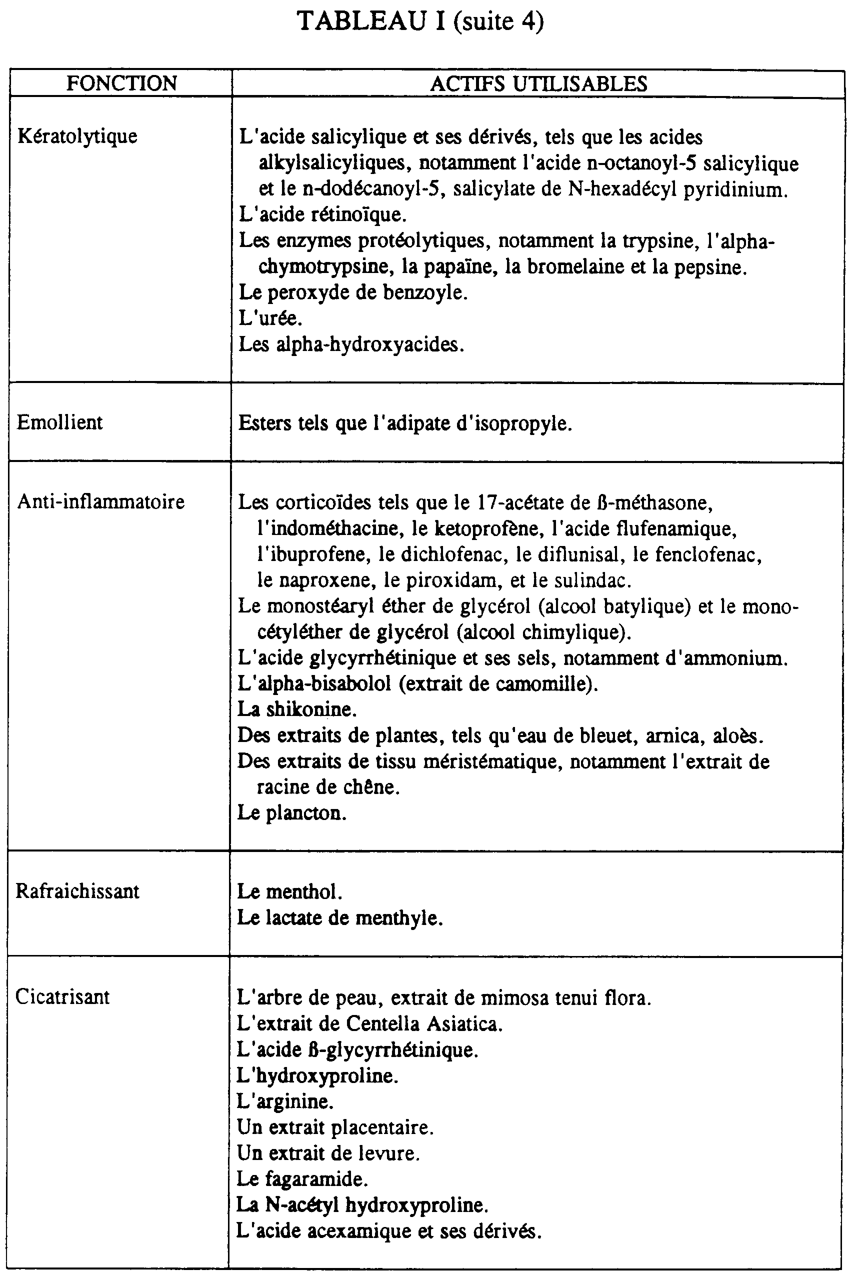

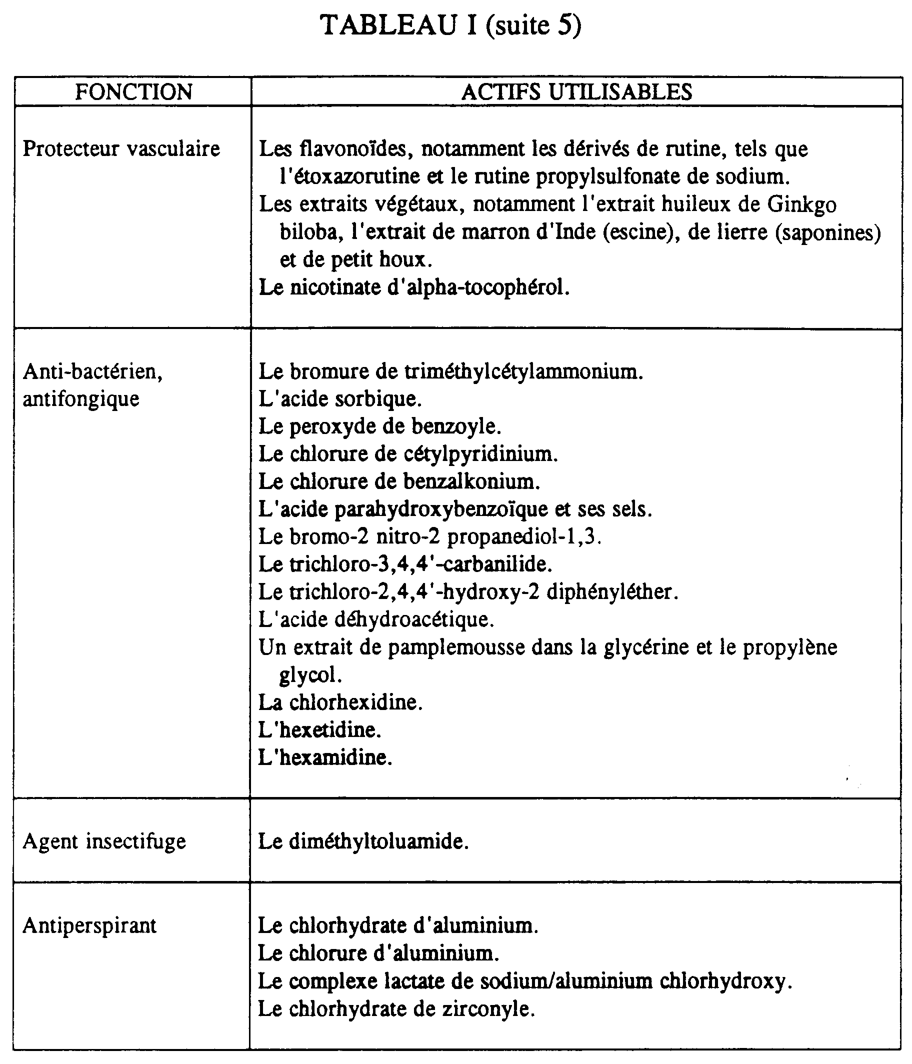

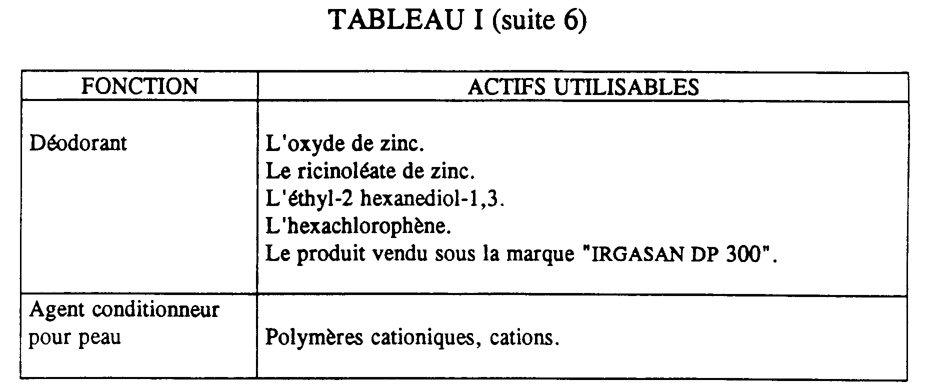

Les compositions selon l'invention peuvent être préparées par tout mode de préparation connu pour la préparation des vésicules de lipide amphiphile. Divers modes de préparation sont, par exemple, décrits dans "Les liposomes en biologie cellulaire et pharmacologie", Editions INSERM/John Libbey Emotext, 1987, pages 6 à 18.

Les compositions vésiculaires selon l'invention sont préparées, de préférence, par l'un des deux procédés connus ci-après définis :
1°- Selon le premier procédé, décrit dans le brevet FR-B-2 315 991, on forme une phase lamellaire plane par introduction de la phase aqueuse à encapsuler dans la phase lipidique membranaire liquide, à une température légèrement supérieure à la température de fusion des lipides, on ajoute ensuite à la phase lamellaire obtenue une phase aqueuse de dispersion, qui peut être identique ou non à la phase aqueuse à encapsuler et on agite énergiquement, par exemple mécaniquement, pour obtenir le passage de la phase lamellaire plane à une dispersion de vésicules.
2°- Selon le deuxième procédé, on procède en deux stades :
   - dans un premier stade, on prépare la phase lipidique devant former la membrane des vésicules, par dissolution dans un solvant des constituants de la phase lipidique membranaire et, éventuellement d'un (ou plusieurs) composé(s) cosmétiquement et/ou pharmaceutiquement actif(s) liposoluble(s) et on évapore le solvant sous pression réduite ;
   - dans un second stade, on ajoute la phase aqueuse de dispersion et on homogénéise le mélange par un moyen mécanique du type secouage et/ou ultrasons, pour obtenir la dispersion de vésicules.

L'homogénéisation est effectuée à une température comprise entre 10°C et 120°C, de préférence entre 30 et 80°C.

Les compositions selon l'invention peuvent se présenter sous la forme de crèmes, de gels, de lotions ou de sérums en ajoutant, de façon connue, dans la phase aqueuse de dispersion, des additifs de formulation n'ayant ni activité cosmétique, ni activité dermopharmaceutique propre. Parmi ces additifs, on peut citer les gélifiants, les polymères, les conservateurs, les colorants et les parfums.

Pour mieux faire comprendre l'objet de l'invention, on va en décrire maintenant, à titre d'exemples purement illustratifs et non limitatifs, plusieurs modes de mise en oeuvre.

### EXEMPLE 1 :

On a étudié le pouvoir anti-oxydant de l'eau de LA ROCHE-POSAY dans une composition vesiculaire, d'une part, et à l'état non-vésiculé, d'autre part. La composition vesiculaire est celle définie à l'exemple 2 ci-après.

Le pouvoir anti-oxydant a été étudié sur des cultures cellulaires de fibroblastes humains. Les cellules sont cultivées pendant 21 jours, d'une part, dans l'eau minérale non vésiculée et, d'autre part, dans l'eau minérale vésiculée. Après adhésion des cellules aux falcons on les soumet à un stress par des radiations ultraviolettes UVB. à la dose de 0,1 J/cm² pendant 2 minutes, selon le protocole décrit par RICHARD et autres, dans "NOUVELLES DERMATOLOGIQUES", Mars 1990, volume 9, n° 3.

On a dosé l'activité enzymatique "Glutathion Peroxydase" (GPX) par la méthode de GUNTZLER modifiée (voir GUNTZLER W.A. et autres, Z. Klin Chem. Klin. Biochem., 12, 444 448 ; 1974) et l'activité enzymatique "Superoxyde Dismutase" (SOD) par la méthode de MARKLUND (voir MARKLUND S. et MARKLUND G., Eur. J. Biochem., 47, 469-474 ; 1974) ; on a évalué la peroxydation lipidique par dosage du malondialdéhyde par la méthode à l'acide thiobarbiturique (voir DOUSSET et autres, Clin. Chim. Acta, 129, 319-322 ; 1983). L'activité GPX rend compte de la capacité cellulaire a épurer les peroxydes apparus à l'état basal. L'activité SOD rend compte également de cette capacité. La teneur en malondialdéhyde doit, au cours du temps, diminuer pour rendre compte d'une activité antiradicalaire liée à l'eau, vésiculée ou non.

Les résultats obtenus sont consignes dans le tableau ci-dessous (moyenne sur trois souches de fibroblastes humains). L'activité GPX est exprimée en micromoles/min/g de protéine ; l'activité SOD est exprimée en micromoles/mg de protéine ; le malondialdéhyde est exprimé en micromoles/g de protéine.

| | Eau ROCHE-POSAY non vésiculée | Eau ROCHE-POSAY vésiculée |
|---|---|---|
| Activité GPX | 156 | 186 |
| Activité SOD | 2.0 | 2.6 |
| Malondialdéhyde | 1.1 | 0.9 |

Les résultats obtenus montrent que l'activité GPX et SOD, qui sont deux maillons de la chaîne de détoxification radicalaire ont leurs activités potentialisées en présence d'eau de LA ROCHE-POSAY, encapsulée dans des vésicules de lipides non-ioniques. De façon corrélée, la diminution du malondialdéhyde rend compte de cette activité de détoxification. Le fait de vésiculer l'eau minérale dans des vésicules de lipides non-ioniques potentialise les effets de l'eau minérale, notamment ses effets antiradicalaires.

On a constaté le même résultat en remplaçant l'eau de LA ROCHE-POSAY par de l'eau de "VITTEL Grande Source".

### EXEMPLE 2 :

La formulation de cet exemple, comme celle de l'exemple 3, a été obtenue selon le procédé défini dans le brevet FR-B- 2 315 991, la fision de lipides ayant été obtenue à 90°C et la phase aqueuse de dispersion étant la même que la phase aqueuse à encapsuler.

On a de la sorte réalisé une composition vésiculaire répondant à la formulation suivante :

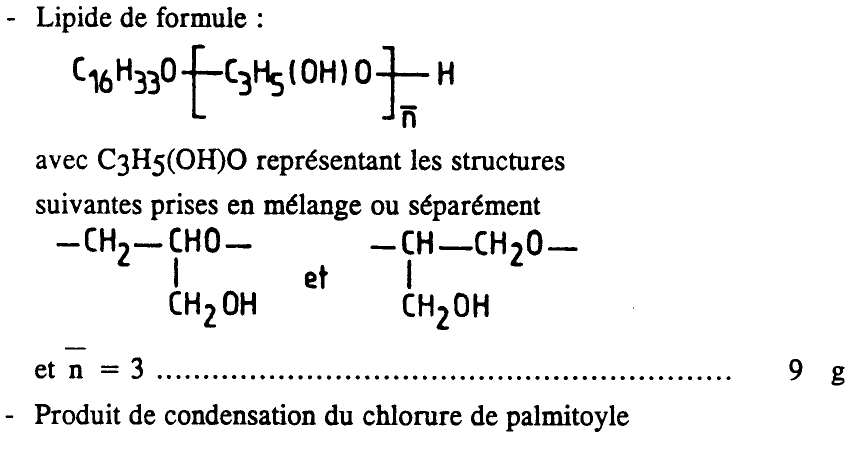

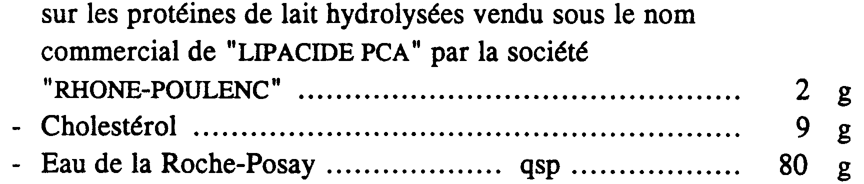

Les vésicules obtenues ont un diamètre moyen de 0,3 µm (homogénéisation effectuée à 45°C).

### EXEMPLE 3 :

Selon le procédé de l'exemple 2, on réalise une composition vesiculaire ayant la formulation suivante :

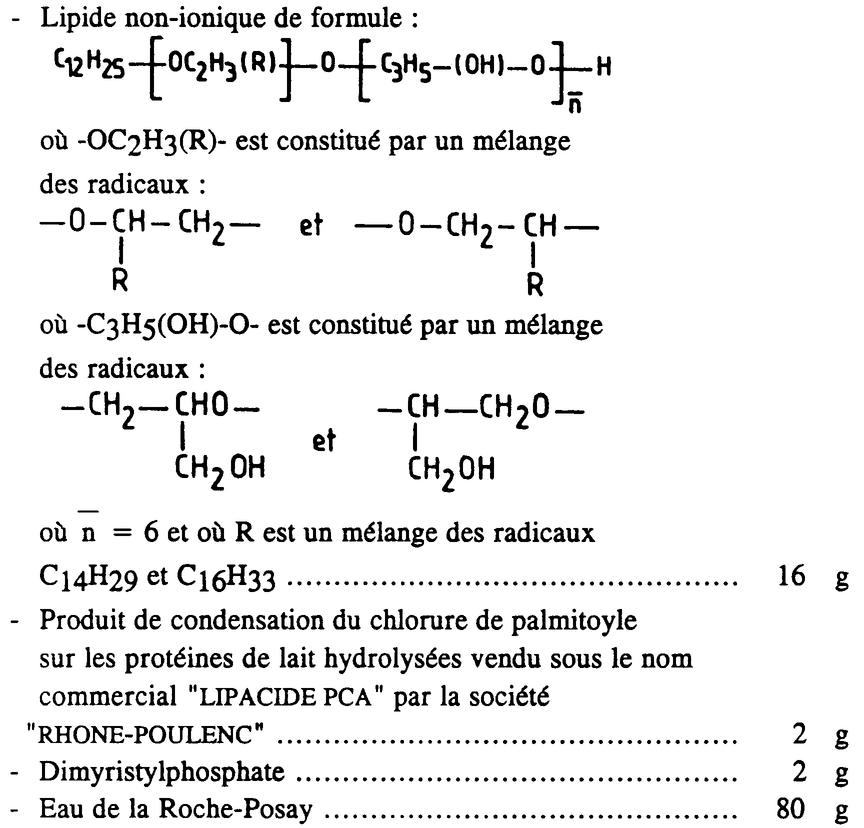

Les vésicules obtenues ont un diamètre moyen de 0,2 µm (homogénéisation effectuée à 45°C).

### EXEMPLE 4 :

La composition de cet exemple, de même que celles des exemples 5 et 6, a été obtenue en dissolvant la phase lipidique devant former la membrane des vésicules, dans un mélange dichlorométhane/méthanol (80/20), à raison de 10 cm³ de solvant par gramme de mélange de lipides, et en évaporant le solvant sous pression réduite ; après quoi, on ajoute l'eau minérale et on homogénéise le mélange par secouage à environ 45°C.

On a ainsi réalisé une composition vesiculaire correspondant à la formulation suivante :

| | |
|---|---|
| - Ester de l'acide stéarique avec un polyglycérol contenant en moyenne 4 unités glycérol, vendu sous le nom commercial "TETRAGLYNE 1s" par la société "NIKKO" (nom CTFA : Polyglycéryl-4 stéarate) | 10 g |
| - Cholestérol | 10 g |
| - Eau "Vittel Grande Source" | 80 g |

On obtient une dispersion, dont les vésicules ont un diamètre moyen de 0,25 µm.

### EXEMPLE 5 :

On réalise selon le procédé de l'exemple 4, une composition vésiculaire répondant à la formulation suivante :

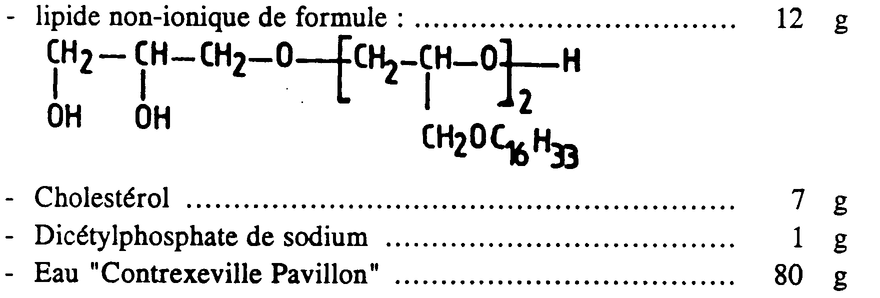

On obtient des vésicules de diamètre moyen de 0,3 µm.

### EXEMPLE 6 :

On réalise selon le procédé de l'exemple 4, une composition vésiculaire répondant à la formulation suivante :

| | |
|---|---|
| - 2-Oléoylamino 1,3-octadécanediol (mélange érythro thréo = 75/25) décrit dans la demande de brevet FR-A- 91 02091 | 3 g |
| - Cholestérol | 4 g |
| - Dicétylphosphate de sodium | 3 g |
| - Eau de Vittel qsp | 100 g |

On obtient des vésicules de diamètre moyen de 0,3 µm.

Les compositions correspondant aux exemples 2 à 6 peuvent être utilisées directement à raison de deux applications par jour pendant 15 jours pour calmer les irritations d'une peau fragile.

Mais les compositions des exemples 2 à 6 peuvent également être utilisées comme compositions vésiculaires de base servant à la formulation d'un quelconque des exemples 7 à 11.

### EXEMPLE 7 : Gel

| | |
|---|---|
| - Composition vésiculaire selon l'exemple 4 | 40 g |
| - Acide polyacrylique réticulé vendu sous le nom commercial de "CARBOPOL 940" par la société "GOODRICH" | 0,4 g |
| - Triéthanolamine | 0,5 g |
| - Conservateurs qs Eau "Vittel Grande Source" qsp | 100 g |

On utilise cette composition à raison de deux applications par jour pendant 15 jours pour le traitement des prurits et pour calmer les irritations de la peau.

### EXEMPLE 8 : Lait

| | |
|---|---|
| - Composition vésiculaire selon l'exemple 3 | 40 g |
| - Polyisobutène hydrogéné vendu par la société "NICHIYU" sous la dénomination commerciale de "PARLEAM" | 6 g |
| - Acide polyacrylique réticulé vendu sous le nom commercial de "CARBOPOL 940" par la société "GOODRICH" | 0,2 g |
| - Triéthanolamine | 0,25 g |
| - Conservateurs qs | |
| - Eau de LA Roche-Posay qsp | 100 g |

On utilise cette composition dans le traitement de la scérose cutanée en application quotidienne jusqu'à amélioration des symptomes.

### EXEMPLE 9 : Lotion

| | |
|---|---|
| - Composition vésiculaire selon l'exemple 5 | 32 g |
| - Glycérol | 3 g |
| - Propylène glycol | 3 g |
| - Acide polyacrylique réticulé vendu sous le nom commercial de "CARBOPOL 940" par la société "GOODRICH" | 0,15 g |
| - Triéthanolamine | 0,2 g |
| - Conservateurs qs | |
| - Eau "Contrexeville Pavillon" qsp | 100 g |

On utilise cette composition comme celle de l'exemple 8.

### EXEMPLE 10 : Crème

| | |
|---|---|
| - Composition vésiculaire selon l'exemple 2 | 40 g |
| - Glycérol | 3 g |
| - Huile de vaseline fluide | 20 g |
| - Acide polyacrylique réticulé vendu sous le nom commercial de "CARBOPOL 940" par la société "GOODRICH" | 0,4 g |
| - Triéthanolamine | 0,5 g |
| - Conservateurs, parfum qs | |
| - Eau de LA Roche-Posay qsp | 100 g |

On utilise cette composition comme celle de l'exemple 7.

### EXEMPLE 11 : Crème

| | |
|---|---|
| - Composition vésiculaire selon l'exemple 6 | 89 g |
| - Acide polyacrylique réticulé vendu sous le nom commercial de "CARBOPOL 940" par la société "GOODRICH" | 0,5 g |
| - Triéthanolamine | 0,5 g |
| - Huile de vaseline fluide | 10 g |

On utilise cette composition comme celle de l'exemple 10.

### EXEMPLE 12 :

Selon le procédé de l'exemple 2, on prépare successivement deux dispersions vésiculaires.

### A) Première dispersion vésiculaire

| | |
|---|---|
| - lipide non-ionique de l'exemple 2 | 1,8 g |
| - Cholestérol | 1,8 g |
| - Lipacide caséinique | 0,4 g |
| - Glycérol | 2,0 g |
| - Eau de La Roche Posay | 23,0 g |

### B) Deuxième dispersion vésiculaire

| | |
|---|---|
| - Lécithine de soja hydrogénée vendue par la société "NIKKO" sous la dénomination commerciale "LECINOL S 10" | 2,4 g |
| - Cholestérol | 1,2 g |
| - Sel de sodium de glutamate de suif hydrogéné vendu sous la dénomination commerciale "ACYLGLUTAMATE HS 11" par la société "AJINOMOTO" | 0,4 g |
| - Glycérol | 4,0 g |
| - Eau de La Roche Posay | 21,0 g |

On mélange les deux dispersions vésiculaires et on ajoute 25 g de polyisobutène hydrogéné vendu par la société "NICHIYU" sous la dénomination commerciale de "PARLEAM". On homogénéise le tout à l'aide d'un ultradisperseur. On ajoute alors la formulation suivante :

| | |
|---|---|
| - Acide polyacrylique réticulé vendu sous le nom commercial de "CARBOPOL 940" par la société "GOODRICH" | 0,42 g |
| - Triéthanolamine | 0,40 g |
| - Conservateurs qs | |
| - Eau de La Roche Posay qsp | 100 g |

On utilise la composition ainsi obtenue comme celle de l'exemple 10.

### EXEMPLE 13 :

Selon le procédé de l'exemple 2, on prépare la composition suivante :

| | |
|---|---|
| - Lécithine de soja hydrogénée vendue par la société "NIKKO" sous la dénomination commerciale "LECINOL S 10" | 12 g |
| - Phytostérol oxyéthyléné à 5 moles d'OE vendu par la société "HENKEL" sous la dénomination commerciale de "GENEROL 122 E 5" | 8 g |
| - Acide polyacrylique réticulé vendu sous le nom commercial de "CARBOPOL 940" par la société "GOODRICH" | 0,4 g |
| - Triéthanolamine | 0,5 g |
| - Conservateurs, parfums qs | |
| - Eau de La Roche Posay qsp | 100 g |

On utilise cette composition comme celle obtenue à l'exemple 10.

### EXEMPLE 14 :

On prépare par le procédé décrit dans l'exemple 4 une crème de soin des ongles ayant la composition suivante :

| | |
|---|---|
| - Ester de l'acide stéarique avec un polyglycérol contenant en moyenne 4 unités glycol, vendu sous le nom commercial "TETRAGLYNE 1S" par la société "NIKKO" (nom CFTA : Polyglycéryl-4 stérate) | 20,0 g |
| - Cholestérol | 20,0 g |
| - Acide polyacrylique réticulé vendu sous le nom commercial "CARBOPOL 940" par la société "GOODRICH" | 0,5 g |
| - Triéthanolamine | 0,4 g |
| - Eau de "VITTEL GRANDE SOURCE" qsp | 100 g |

On observe une amélioration de l'hydratation et de la plasticité d'ongles cassants après application quotidienne de cette composition pendant 2 mois.

## Revendications

1. Composition pour le traitement topique de la peau ou des ongles comportant dans une phase aqueuse de dispersion, des vésicules délimitées par une membrane de phase lipidique contenant au moins un lipide amphiphile, lesdites vésicules contenant une phase encapsulée au moins partiellement constituée d'au moins une eau minérale formant un actif pour le traitement de la peau, caractérisée par le fait que la phase lipidique membranaire des vésicules contient au moins un lipide amphiphile non-ionique choisi dans le groupe formé par :
(1) les dérivés du glycérol, linéaires ou ramifiés, de formule 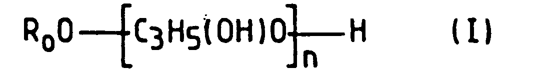 formule (I) dans laquelle :
. -C₃H₅(OH)O-
est représenté par les structures suivantes prises en mélange ou séparément :
-CH₂CHOHCH₂O- , 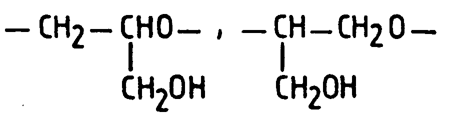
. n est une valeur statistique moyenne comprise entre 1 et 6 ou bien n = 1 ou 2, auquel cas -C₃H₅(OH)O- est représenté par la structure -CH₂-CHOH-CH₂O ;
. Rₒ représente :
(a) une chaîne aliphatique, linéaire ou ramifiée saturée ou insaturée, contenant de 12 à 30 atomes de carbone ; ou des radicaux hydrocarbonés des alcools de lanoline ; ou les restes d'alpha-diols à longue chaîne ;
(b) un reste R₁CO, où R₁ est un radical aliphatique, linéaire ou ramifié, en C₁₁-C₂₉ ;
(c) un reste R₂⁅OC₂H₃(R₃)⁆
où :
. R₂ peut prendre la signification (a) ou (b) donnée pour Rₒ ;
. -OC₂H₃(R₃)- est représenté par les structures suivantes, prises en mélange ou séparément : 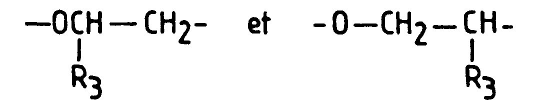
où R₃ prend la signification (a) donnée pour Rₒ ;
(2) les éthers de polyglycérol, linéaires ou ramifiés, comportant deux chaînes grasses ;
(3) les diols à chaîne grasse ;
(4) les alcools gras, oxyéthylénés ou non, les stérols et les phytostérols, oxyéthylénés ou non ;
(5) les éthers et esters de polyols, oxyéthylénés ou non, l'enchaînement des oxydes d'éthylène pouvant être linéaire ou cyclique ;
(6) les éthers et esters de mono ou polysaccharides et les éthers et les esters de glucose ;
(7) les hydroxylamides représentés par la formule : 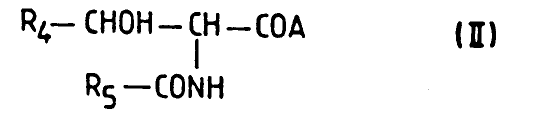 formule (II) dans laquelle :
- R₄ désigne un radical alkyle ou alcényle en C₇-C₂₁ ;
- R₅ désigne un radical hydrocarboné, saturé ou insaturé, en C₇-C₃₁ ;
- COA désigne un groupement choisi parmi les deux groupements suivants :
. un reste 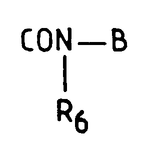 où :
. B est un radical alcoyle dérivé d'amines primaires ou secondaires, mono- ou polyhydroxylées ; et
. R₆ désigne un atome d'hydrogène ou un radical méthyle, éthyle ou hydroxyéthyle ; et
. un reste -COOZ, où Z représente le reste d'un polyol en C₃-C₇.
(8) les céramides naturels ou de synthèse ;
(9) les dihydroxyalkylamines, les amines grasses oxyéthylénées ;
(10) les dérivés de glycérol répondant à la formule : 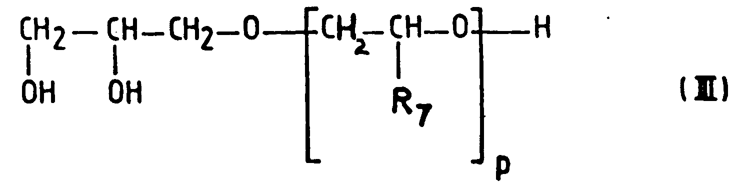 formule (III) dans laquelle R₇ représente un radical alkyle linéaire en C₁₄ à C₁₈ ou un groupement -CH₂A dans lequel A est -OR₁₄, R₁₄ représentant un radical alkyle linéaire en C₁₀-C₁₈ et p représente une valeur statistique moyenne supérieure à 1 et au plus égale à 3 et, en outre lorsque R₇ = -CH₂A, p peut également représenter une valeur réelle égale à 2.

2. Composition selon la revendication 1, caractérisée par le fait que le(s) lipide(s) amphiphile(s) de la phase lipidique membranaire est (sont tous) non-ionique(s).

3. Composition selon la revendication 1, caractérisée par le fait que les lipides amphiphiles de la phase lipidique membranaire sont constitués par un mélange de lipide(s) ionique(s) et de lipide(s) non-ionique(s).

4. Composition selon la revendication 3, caractérisée par le fait que le(s) lipide(s) amphiphile(s) ionique(s) est (sont) choisi(s) dans le groupe formé par :
(1) les lipides amphiphiles anioniques suivants :
• les phospholipides naturels, les phospholipides modifiés par voie chimique ou enzymatique, et les phospholipides de synthèse ;
• les composés anioniques de formule 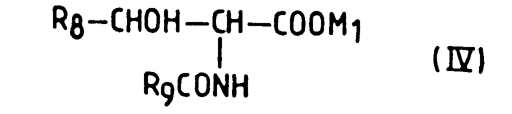 formule (IV) dans laquelle :
. R₈ représente un radical alkyle ou alcényle en C₇-C₂₁
. R₉ représente un radical hydrocarboné, saturé ou insaturé, en C₇-C₃₁, et
. M₁ représente H, Na, K, NH₄ ou un ion ammonium substitué dérivé d'une amine ;
• les esters phosphoriques d'alcools gras, notamment le dicétylphosphate et le dimyristylphosphate sous forme d'acides ou de sels alcalins ; l'acide heptylnonylbenzène sulfonique ; le sulfate de cholestérol acide ou le phosphate de cholestérol acide, ainsi que leurs sels alcalins ; les lysolécithines ; les alkylsulfates, notamment le cétylsulfate de sodium ; les gangliosides ;
(2) les lipides amphiphiles cationiques suivants :
• les composés cationiques ayant la formule : 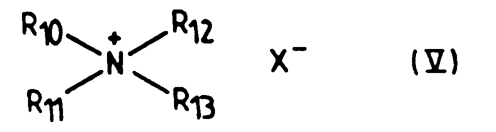 formule (V) dans laquelle R₁₀ et R₁₁, identiques ou différents, représentent des radicaux alkyle en C₁₂ - C₂₀ et R₁₂ et R₁₃, identiques ou différents, des radicaux alkyle en C₁ - C₄ ;
• les amines à longue chaîne et leurs dérivés ammonium quaternaire ; les esters d'aminoalcools à longue chaîne et leurs sels et dérivés ammonium quaternaire ; et
• les lipides polymérisables.

5. Composition selon l'une des revendications 1 à 4, caractérisée par le fait que la phase aqueuse de dispersion est choisie dans le groupe formé par l'eau et/ou l'eau minérale et les mélanges d'eau et/ou d'eau minérale avec au moins un alcool en C₁-C₇ et/ou un polyol d'alkyle en C₁-C₅.

6. Composition selon l'une des revendications 1 à 5, caractérisée par le fait que la phase aqueuse de dispersion contient une dispersion de gouttelettes d'un liquide non miscible à l'eau.

7. Composition selon la revendication 6 caractérisée par le fait que le liquide non miscible à l'eau qui constitue les gouttelettes dans la phase de dispersion est choisi dans le groupe formé par les huiles animales ou végétales, les huiles essentielles naturelles ou synthétiques, les hydrocarbures, les carbures halogénés, les silicones, les esters d'un acide minéral et d'un alcool, les éthers et les polyéthers.

8. Composition selon l'une des revendications 1 à 7, caractérisée par le fait que la phase lipidique totale de la dispersion représente entre 0,01 % et 50 % en poids par rapport au poids total de la dispersion.

9. Composition selon l'une des revendications 1 à 8, caractérisée par le fait que le(s) lipide(s) amphiphile(s) représente(nt) entre 10 et 95 % en poids par rapport au poids total de la phase lipidique.

10. Composition selon l'une des revendications 1 à 9, caractérisée par le fait que les vésicules ont des dimensions comprises entre 20 et 3 000 nm.

11. Composition selon l'une des revendications 1 à 10, caractérisée par le fait qu'elle contient, outre l'eau minérale, au moins un actif à action cosmétique et/ou pharmaceutique.

12. Composition selon la revendication 11, caractérisée par le fait que la phase aqueuse de dispersion contient au moins un composé hydrosoluble cosmétiquement et/ou pharmaceutiquement actif.

13. Composition selon la revendication 11, caractérisée par le fait que la phase aqueuse encapsulée contient au moins un compose hydrosoluble cosmétiquement et/ou pharmaceutiquement actif.

14. Composition selon la revendication 11, caractérisée par le fait que la phase lipidique des vésicules contient au moins un composé liposoluble cosmétiquement et/ou pharmaceutiquement actif.

15. Composition selon l'une des revendications 1 à 14, caractérisée par le fait qu'elle comprend au moins un additif de formulation assurant sa présentation sous forme de gel, de lotion, de sérum ou de crème.

16. Composition selon l'une des revendications 1 à 15, caractérisée par le fait que l'eau minérale contient au moins un oligoélément et de la silice.

17. Composition selon la revendication 16, caractérisée par le fait que la teneur en silice dans l'eau minérale est comprise entre 1 et 150 mg/l.

18. Composition selon l'une des revendications 1 à 17, caractérisée par le fait qu'elle contient des vésicules dont la phase lipidique membranaire est constituée d'un ou plusieurs lipide(s) amphiphile(s) ionique(s).

## Claims

1. Composition for the topical treatment of the skin or the nails comprising, in an aqueous dispersing phase, vesicles delimited by a membrane consisting of a lipid phase containing at least one amphiphilic lipid, the said vesicles containing an encapsulated phase at least partially consisting of at least one mineral water forming an active ingredient for the treatment of the skin, characterised by the fact that the membrane lipid phase of the vesicles contains at least one non-ionic amphiphilic lipid chosen from the group formed by:
(1) the linear or branched glycerol derivatives of formula 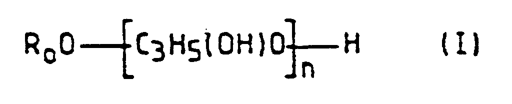 formula (I) in which:
-C₃H₅(OH)O-
is represented by the following structures taken together or separately:
-CH₂CHOHCH₂O- , 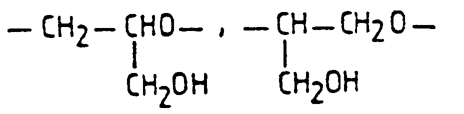
. n is a mean statistical value between 1 and 6 or alternatively n = 1 or 2, in which case -C₃H₅(OH)O- is represented by the structure -CH₂-CHOH-CH₂O;
. R₀ represents:
(a) a saturated or unsaturated, linear or branched aliphatic chain containing 12 to 30 carbon atoms; or hydrocarbon radicals of lanolin alcohols; or long-chain alpha-diol residues;
(b) an R₁CO residue, where R₁ is a linear or branched C₁₁-C₂₉ aliphatic radical;
(c) a residue R₂⁅OC₂H₃(R₃)⁆
where:
. R₂ may have the meaning (a) or (b) given for R₀;
. -OC₂H₃(R₃)- is represented by the following structures, taken together or separately: 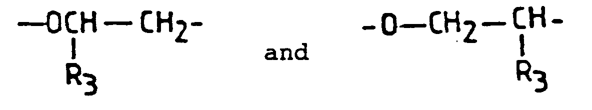
where R₃ has the meaning (a) given for R₀,
(2) linear or branched polyglycerol ethers containing two fatty chains;
(3) fatty chain diols;
(4) oxyethylenated or nonoxyethylenated fatty alcohols, and oxyethylenated or nonoxyethylenated sterols and phytosterols;
(5) oxyethylenated or nonoxyethylenated polyol ethers and esters, it being possible for the ethylene oxide linkage to be linear or cyclic;
(6) mono- or polysaccharide ethers and esters, and glucose ethers and esters;
(7) hydroxyamides represented by the formula: 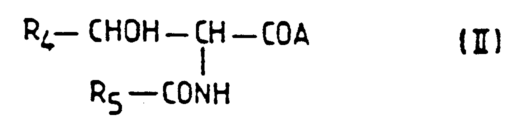 formula (II) in which:
- R₄ denotes a C₇-C₂₁ alkyl or alkenyl radical;
- R₅ denotes a saturated or unsaturated C₇-C₃₁ hydrocarbon radical;
- COA denotes a group chosen from the following two groups:
. a residue 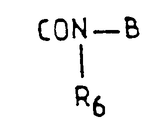 where:
. B is an alkyl radical derived from mono- or polyhydroxylated primary or secondary amines; and
. R₆ denotes a hydrogen atom or a methyl, ethyl or hydroxyethyl radical; and
. a residue -COOZ, where Z represents the residue of a C₃-C₇ polyol.
(8) natural or synthetic ceramides;
(9) dihydroxyalkylamines, oxyethylenated fatty amines;
(10) glycerol derivatives corresponding to the formula: 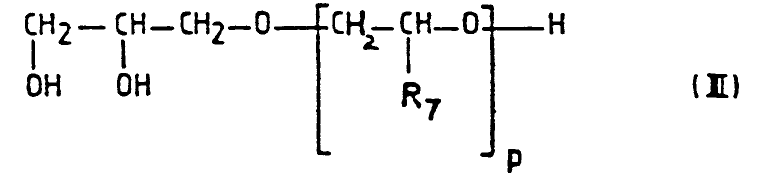 formula (III) in which R₇ represents a linear C₁₄ to C₁₈ alkyl radical or a -CH₂A group in which A is -OR₁₄, R₁₄ representing a linear C₁₀-C₁₈ alkyl radical, and p represents a mean statistical value greater than 1 and at most equal to 3 and, in addition, when R₇ = -CH₂A, p may also represent a real value equal to 2.

2. Composition according to Claim 1, characterised by the fact that the amphiphilic lipid(s) of the membrane lipid phase is (are all) non-ionic.

3. Composition according to Claim 1, characterised by the fact that the amphiphilic lipids of the membrane lipid phase consist of a mixture of ionic lipid(s) and non-ionic lipid(s).

4. Composition according to Claim 3, characterised by the fact that the ionic amphiphilic liquid(s) is (are) chosen from the group formed by:
(1) the following anionic amphiphilic lipids:
• natural phospholipids, phospholipids modified chemically or enzymatically, and synthetic phospholioids;
• anionic compounds of formula 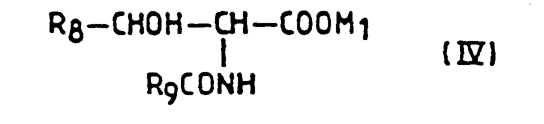 formula (IV) in which:
. R₈ represents a C₇-C₂₁ alkyl or alkenyl radical
. R₉ represents a saturated or unsaturated C₇-C₃₁ hydrocarbon radical, and
. M₁ represents H, Na, K, NH₄ or a substituted ammonium ion derived from an amine;
• phosphoric esters of fatty alcohols especially dicetyl phosphate and dimyristyl phosphate in the form of acids or alkali metal salts; heptyl nonyl benzene sulfonic acid; cholesterol acid sulphate or cholesterol acid phosphate as well as their alkali metal salts; lysolecithins; alkyl sulfates especially sodium cetyl sulfate; gangliosides;
(2) the following cationic amphiphilic lipids:
• the cationic compounds having the formula: 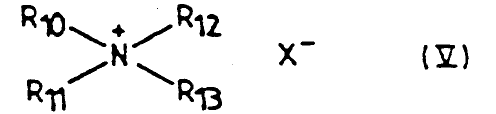 formula (V) in which R₁₀ and R₁₁, which are identical or different, represent C₁₂-C₂₀ alkyl radicals, and R₁₂ and R₁₃, which are identical or different, represent C₁-C₄ alkyl radicals;
• long-chain amines and their quaternary ammonium derivatives; esters of long-chain amino alcohols and their salts and quaternary ammonium derivatives; and
• polymerisable lipids.

5. Composition according to one of Claims 1 to 4, characterised by the fact that the aqueous dispersing phase is chosen from the group formed by water and/or mineral water and mixtures of water and/or mineral water with at least one C₁-C₇ alcohol and/or one C₁-C₅ alkyl polyol.

6. Composition according to one of Claims 1 to 5, characterised by the fact that the aqueous dispersing phase contains a dispersion of droplets of a water-immiscible liquid.

7. Composition according to Claim 6, characterised by the fact that the water-immiscible liquid which constitutes the droplets in the dispersing phase is chosen from the group formed by animal or vegetable oils, natural or synthetic essential oils, hydrocarbons, halogenated carbons, silicones, esters of an inorganic acid and an alcohol, ethers and polyethers.

8. Composition according to one of Claims 1 to 7, characterised by the fact that the total lipid phase of the dispersion represents between 0.01% and 50% by weight relative to the total weight of the dispersion.

9. Composition according to one of Claims 1 to 8, characterised by the fact that the amphiphilic lipid(s) represent(s) between 10 and 95% by weight relative to the total weight of the lipid phase.

10. Composition according to one of Claims 1 to 9, characterised by the fact that the size of the vesicles is between 20 and 3000 nm.

11. Composition according to one of Claims 1 to 10, characterised by the fact that it contains, in addition to the mineral water, at least one active ingredient with cosmetic and/or pharmaceutical action.

12. Composition according to Claim 11, characterised by the fact that the aqueous dispersing phase contains at least one cosmetically and/or pharmaceutically active water-soluble compound.

13. Composition according to Claim 11, characterised by the fact that the aqueous encapsulated phase contains at least one cosmetically and/or pharmaceutically active water-soluble compound.

14. Composition according to Claim 11, characterised by the fact that the lipid phase of the vesicles contains at least one cosmetically and/or pharmaceutically active fat-soluble compound.

15. Composition according to one of Claims 1 to 14, characterised by the fact that it comprises at least one formulation additive allowing its presentation in the form of a gel, lotion, serum or cream.

16. Composition according to one of Claims 1 to 15, characterised by the fact that the mineral water contains at least one trace-element and silica.

17. Composition according to Claim 16, characterised by the fact that the silica content in the mineral water is between 1 and 150 mg/l.

18. Composition according to one of Claims 1 to 17, characterised by the fact that it contains vesicles whose membrane lipid phase is composed of one or more ionic amphiphilic lipids.

## Patentansprüche

1. Mittel zur topischen Behandlung der Haut oder der Nägel, das in einer wäßrigen Dispersionsphase Vesikel umfaßt, die durch eine Membran aus einer wenigstens ein amphiphiles Lipid enthaltenden Lipidphase begrenzt sind, wobei die Vesikel eine Phase eingekapselt enthalten, bei der es sich wenigstens zum Teil um wenigstens ein Mineralwasser handelt, das einen Wirkstoff zur Behandlung der Haut bildet, dadurch gekennzeichnet, daß die Lipidphase der Vesikelmembran wenigstens ein nicht-ionisches amphiphiles Lipid enthält, das ausgewählt ist unter:
(1) geradkettigen oder verzweigten Glycerinderivaten der Formel 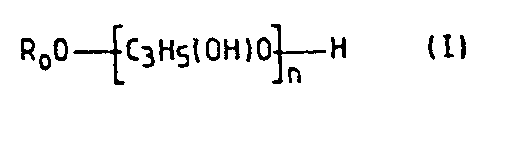 worin:
• C₃H₅(OH)O-
für einen der folgenden Reste oder ein Gemisch dieser Reste steht:
-CH₂CHOHCH₂O- , 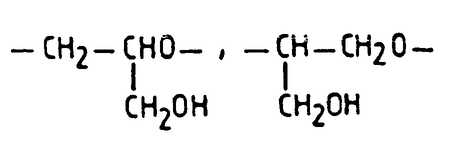
• n einen statistischen Mittelwert von 1 bis 6 bedeutet oder n = 1 oder 2, wenn -C₃H₅(OH)O- für den Rest -CH₂-CHOH-CH₂O steht;
• R₀ bedeutet:
(a) eine geradkettige oder verzweigte, gesättigte oder ungesättigte aliphatische Kette mit 20 bis 30 Kohlenstoffatomen; oder die Kohlenwasserstoffreste des Lanolinalkohols, oder die Reste langkettiger alpha-Diole;
(b) einen Rest R₁CO, worin R₁ einen geradkettigen oder verzweigten aliphatischen C₁₁-C₂₉-Rest bedeutet;
(c) einen Rest R₂⁅OC₂H₃(R₃)⁆
worin:
• R₂ die für R₀ angegebenen Bedeutungen (a) oder (b) annehmen kann;
• -OC₂H₃(R₃)- für einen der folgenden Reste oder ein Gemisch dieser Reste steht: 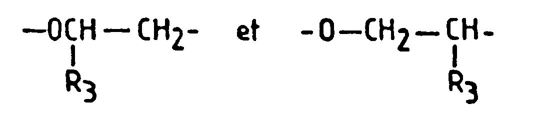
worin R₃ die für R₀ angegebene Bedeutung (a) besitzt;
(2) linearen oder verzweigten Polyglycerinäthern mit zwei Fettketten;
(3) Diolen mit einer Fettkette;
(4) oxyethylenierten oder nicht-oxyethylenierten Fettalkoholen, Sterolen oder oxyethylenierten oder nichtoxyethylenierten Phytosterolen;
(5) Oxyethylenierten oder nicht-oxyethylenierten Polyoläthern und Polyolestern, wobei die Ethylenoxidkette linear oder cyclisch sein kann;
(6) Äthern und Estern von Mono- oder Polysacchariden und Glucoseäthern und Glucoseestern;
(7) Hydroxyamiden der Formel: 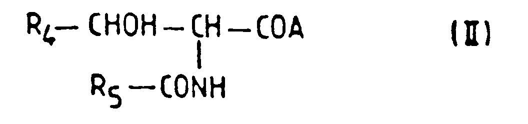 worin:
- R₄ für einen C₇-C₂₁-Alkyl- oder -Alkenylrest steht;
- R₅ für einen gesättigten oder ungesättigten C₇-C₃₁-Kohlenwasserstoffrest steht;
- COA eine Gruppe bedeutet, die ausgewählt ist unter den folgenden Gruppen:
• einem Rest 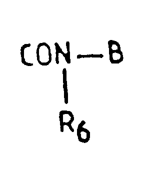 worin:
. B für einen mono- oder polyhydroxylierten von primären oder sekundären Aminen abgeleiteten Alkylrest steht; und
. R₆ für ein Wasserstoffatom oder einen Methyl-, Äthyl- oder Hydroxyäthylrest steht; und
• einem Rest -COOZ, worin Z für einen C₃-C₇-Polyolrest steht;
(8) natürlichen oder synthetischen Ceramiden
(9) Dihydroxyalkylaminen, oxyethylenierten Fettaminen;
(10) Glycerinderivaten der Formel: 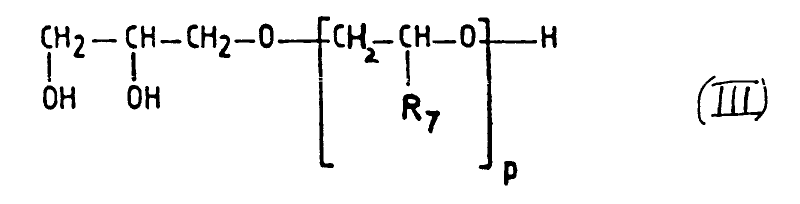 worin R₇ für einen linearen C₁₄-C₁₈-Alkylrest oder eine Gruppe -CH₂A steht, worin A für -OR₁₄ steht, R₁₄ einen linearen C₁₀-C₁₈-Alkylrest bedeutet und P für einen statistischen Mittelwert von größer als 1 und höchstens gleich 3 steht und, wenn R₇ = CH₂A, p außerdem den realen Wert von 2 annehmen kann.

2. Mittel nach Anspruch 1, dadurch gekennzeichnet, daß das amphiphile Lipid (die amphiphilen Lipide) der Lipidmembranphase nicht-ionisch ist (sind).

3. Mittel nach Anspruch 1, dadurch gekennzeichnet, daß die amphiphilen Lipide der Lipidmembranphase eine Mischung eines ionischen Lipids und eines nicht-ionischen Lipids (von ionischen Lipiden und nicht-ionischen Lipiden) ist (sind).

4. Mittel nach Anspruch 3, dadurch gekennzeichnet, daß das ionische amphiphile Lipid (die ionischen amphiphilen Lipide) ausgewählt ist (sind) unter:
(1) den folgenden anionischen amphiphilen Lipiden:
• natürlichen Phospholipiden, auf chemischen oder enzymatischem Weg modifizierten Phospholipiden und synthetischen Phospholipiden;
• anionischen Verbindungen der Formel 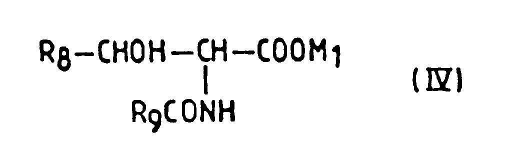 worin:
. R₈ für einen C₇-C₂₁-Alkyl- oder -Alkenylrest steht,
. R₉ für einen gesättigten oder ungesättigten C₇-C₃₁-Kohlenwasserstoffrest und
. M₁ für H, Na, K, NH₄ oder ein für ein substituiertes von einem Amin abgeleitetes Ammoniumion steht;
• Phosphorsäureestern von Fettalkohlen, insbesondere Dicetylphosphat und Dimyristylphosphat in Form der Säure oder der Alkalisalze; Heptylnonylbenzolsulfonsäure; saurem Cholesterinsulfat oder saurem Cholesterinphosphat sowie den Alkalisalzen davon; Lysolecithinen; Alkylsulfaten, insbesondere Natriumcetylsulfat; Gangliosiden;
(2) den folgenden kationischen amphiphilen Lipiden:
• kationischen Verbindungen der Formel: 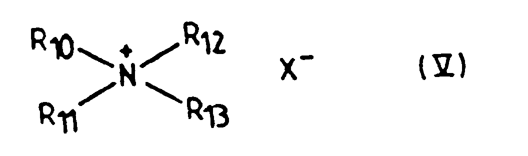 worin R₁₀ und R₁₁, die gleich oder verschieden sind, für einen C₁₂-C₂₀-Alkylrest stehen und R₁₂ und R₁₃, die gleich oder verschieden sind, für einen C₁-C₄-Alkylrest stehen;
• langkettigen Aminen und ihren quaternären Ammoniumderivaten; Estern von Aminoalkoholen mit langer Kette und ihren Salzen und quaternären Ammoniumderivaten und
• polymerisierbaren Lipiden.

5. Mittel nach einem der Ansprüche 1 bis 4, dadurch gekennzeichnet, daß die wäßrige Dispersionsphase ausgewählt ist unter Wasser und/oder Mineralwasser und Mischungen von Wasser und/oder Mineralwasser mit wenigstens einem C₁-C₇-Alkohol und/oder einem C₁-C₅-Alkylpolyol.

6. Mittel nach einem der Ansprüche 1 bis 5, dadurch gekennzeichnet, daß die wäßrige Dispersionsphase eine Dispersion vom Tröpfchen einer mit Wasser nicht-mischbaren Flüssigkeit enthält.

7. Mittel nach Anspruch 6, dadurch gekennzeichnet, daß die mit Wasser nicht-mischbare Flüssigkeit, welche die Tröpfchen in der Dispersionsphase bildet, ausgewählt ist unter tierischen oder pflanzlichen Ölen, natürlichen oder synthetischen essentiellen Ölen, Kohlenwasserstoffen, Halogenkohlenstoffen, Silikonen, Estern aus einer Mineralsäure und einem Alkohol, Äthern und Polyäthern.

8. Mittel nach einem der Ansprüche 1 bis 7, dadurch gekennzeichnet, daß die Gesamtlipidphase der Dispersion 0,01 bis 50 Gew.-%, bezogen auf das Gesamtgewicht der Dispersion, ausmacht.

9. Mittel nach einem der Ansprüche 1 bis 8, dadurch gekennzeichnet, daß das amphiphile Lipid (die amphiphilen Lipide) 10 bis 95 Gew.-%, bezogen auf das Gesamtgewicht der Lipidphase, ausmacht (ausmachen).

10. Mittel nach einem der Ansprüche 1 bis 9, dadurch gekennzeichnet, daß die Vesikel Abmessungen im Bereich von 20 bis 3000 nm aufweisen.

11. Mittel nach einem der Ansprüche 1 bis 10, dadurch gekennzeichnet, daß es zusätzlich zu dem Mineralwasser wenigstens einen kosmetischen und/oder pharmazeutischen Wirkstoff enthält.

12. Mittel nach Anspruch 11, dadurch gekennzeichnet, daß die wäßrige Dispersionsphase wenigstens eine wasserlösliche, kosmetisch und/oder pharmazeutisch wirksame Verbindung enthält.

13. Mittel nach Anspruch 11, dadurch gekennzeichnet, daß die eingekapselte wäßrige Phase wenigstens eine wasserlösliche, kosmetisch und/oder pharmazeutisch wirksame Verbindung enthält.

14. Mittel nach Anspruch 11, dadurch gekennzeichnet, daß die Lipidphase der Vesikel wenigstens eine fettlösliche, kosmetisch und/oder pharmazeutisch wirksame Verbindung enthält.

15. Mittel nach einem der Ansprüche 1 bis 14, dadurch gekennzeichnet, daß es wenigstens ein Formulierungsadditiv für die Bereitstellung in Form eines Gels, einer Lotion, eines Serums oder einer Creme enthält.

16. Mittel nach einem der Ansprüche 1 bis 15, dadurch gekennzeichnet, daß das Mineralwasser wenigstens ein Oligoelement und Siliciumdioxid enthält.

17. Mittel nach Anspruch 16, dadurch gekennzeichnet, daß der Gehalt an Siliciumdioxid im Mineralwasser 1 bis 150 mg/l beträgt.

18. Mittel nach einem der Ansprüche 1 bis 17, dadurch gekennzeichnet, daß es Vesikel enthält, deren Lipidmembranphase durch ein amphiphiles ionisches Lipid oder mehrere amphiphile ionische Lipide gebildet ist.
